(19) Europäisches Patentamt
European Patent Office
Office européen des brevets

(11) Publication number: **0 192 327**
**A2**

# (12) EUROPEAN PATENT APPLICATION

(21) Application number: **86300295.2**

(22) Date of filing: **17.01.86**

(51) Int. Cl.⁴: **A 61 K 31/66**

(30) Priority: **18.01.85 GB 8501374**

(43) Date of publication of application:
**27.08.86 Bulletin 86/35**

(84) Designated Contracting States:
**AT BE CH DE FR GB IT LI LU NL SE**

(71) Applicant: **THE WELLCOME FOUNDATION LIMITED**
**183-193 Euston Road**
**London NW1 2BP(GB)**

(72) Inventor: **St-Clair, Martha Heider**
**312 Seven Oaks Road**
**Durham North Carolina 27704(US)**

(72) Inventor: **Furman, Phillip Allen**
**901 Bluestone Road**
**Durham North Carolina 27713(US)**

(72) Inventor: **Barry, David Walter**
**1810 Lakeshore Drive**
**Chapel Hill North Carolina 27514(US)**

(74) Representative: **Garrett, Michael et al,**
**The Wellcome Foundation Limited Group Patents and Agreements Langley Court**
**Beckenham Kent BR3 3BS(GB)**

(54) **Use of phosphonoformic acid and its esters and salts in the treatment of AIDS.**

(57) The present invention relates to the use of phosphonoformic acid, its esters and salts, in the treatment of acquired immune deficiency syndrome (AIDS).

EP 0 192 327 A2

Croydon Printing Company Ltd

# Antiviral Compounds

The present invention relates to the treatment of a human being diagnosed as being afflicted with acquired immune deficiency syndrome with a compound of formula (I) or a pharmaceutically acceptable salt thereof.

Acquired immune deficiency syndrome (AIDS) is an immunosuppressive or immunodestructive disease that predisposes subjects to fatal opportunistic infections. Characteristically, AIDS is associated with a progressive depletion of T-cells, especially the helper-inducer subset bearing the $OKT^4$ surface maker.

Human T-cell lymphotropic virus III (HTLV-III) has been reproducibly isolated from patients with AIDS or with signs and symptoms that frequently precede AIDS. HTLV-III, unlike HTLV-I or HTLV-II, is cytopathic and appears to preferentially infect and destroy $OKT^4$-bearing T-cells.

While it is presumed that HTLV-III is the aetiologic agent of AIDS, it will be understood that the taxonomy of this virus has yet to be confirmed so, for example, it is also known as lymphadenopathy virus (LAV). Thus, any reference to AIDS refers to the condition and not specifically to an HTLV-III infection.

US Patents Nos. 4,339,445 and 4,536,400 disclose the use of compounds of formula (I) in the treatment of viral infections, including RNA tumour virus infections. Thus, for example, certain animal retroviruses, such as visna virus, fall into this category, as do certain human retroviruses, such as HTLV-I, the causative agent of certain leukaemia conditions in man, wherein T-cells are caused to mutate, and proliferate rapidly. However, HTLV-III is not an RNA tumour virus in that it is not tumour - forming. Infection with HTLV-III results in rapid destruction of T-cells and is, thus, a cytopathic virus.

The present invention provides a compound of formula (I)

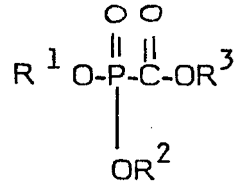

(I)

(wherein $R^1$, $R^2$ and $R^3$ may be the same or different and each is selected from hydrogen, $C_{1-6}$ alkyl, $C_{3-6}$ cycloalkyl, $C_{4-7}$ cycloalkylalkyl, benzyl and phenyl having up to three optional substituents selected from halogen, $C_{1-4}$ alkyl, $C_{1-4}$ alkoxy, $C_{2-7}$ alkoxycarbonyl and $C_{2-7}$ alkylcarbonyl; and pharmaceutically acceptable salts thereof when at least one of $R^1$, $R^2$ and $R^3$ is hydrogen) for use in the treatment or prophylaxis of HTLV-III infections (including AIDS).

The invention further provides the use of a compound of formula (I), and pharmaceutically acceptable salts thereof, in the manufacture of a medicament for the treatment of HTLV-III infections (including AIDS).

The present invention further includes a method for the treatment of a human being diagnosed as being infected with HLTV-III which comprises administering to said human being an effective HLTV-III infection treating amount of a compound of formula (I) or a pharmaceutically acceptable salt thereof.

The present invention further provides a preferred method of treating a human being having been diagnosed as having AIDS or being infected with HLTV-III with a compound of formula (I) wherein $R^1$, and $R^2$ and $R^3$ are all hydrogen or a pharmaceutically acceptable salt thereof.

Preferred pharmaceutically acceptable salts of the compound of formula (I) wherein at least one of $R^1$, $R^2$, and $R^3$ is hydrogen comprise a pharmaceutically acceptable cation in association with an anion of a compound of formula (I). Suitable cations are mono-, di- or trivalent and include $Na^+$, $K^+$, $NH_4^+$, $NR_4^+$ (wherein R is $C_{1-6}$ alkyl), $Ca^{++}$, $Mg^{++}$ $Al^{+++}$, $Ba^{++}$, and $Zn^{++}$.

The compound of formula (I) and the pharmaceutically acceptable salts thereof (hereafter collectively referred to as the active ingedient) may be administered by any suitable route inlcuding oral, rectal, nasal, topical (including buccal and sublingual), vaginal and parenteral (including subcutaneous, intramuscular, intravenous and intradermal). It will be appreciated that the preferred route may vary with, for example, the condition and age of the recipient.

The compounds of formula (I) and their salts as well as pharmaceutical formulations thereof are known and are described, for example, in U.S. Patent Nos. 4,215,113; 4,339,445; and 4,386,081 all of which are incorporated herein by reference.

HDL/OLM/2nd January 1986

In general, a suitable effective dose will be in the range 1.0 to 250 mg per kilogram body weight of recipient per day, preferably in the range of 1 to 100 mg per kilogram body weight per day and most preferably in the range 5 to 40 mg per kilogram body weight per day. (Unless otherwise indicated all weights of active ingredient are calculated as the parent compound of formula (I): for salts thereof the figures would be increased proportionately.) The desired dose is preferably presented as two, three, four or more sub-doses administered at appropriate intervals throughout the day. These sub-doses may be administered in unit dosage forms, for example, containing 10 to 1000 mg, preferably 20 to 500 mg and most preferably 100 to 400 mg of active ingedient per unit dosage form.

A preferred dose is administered to achieve peak plasma concentrations of a compound of formula (I) of from about 100 to about 500 µM. This may be achieved, for example, by the intravenous injection of a 0.1 to 5% solution of the compound of formula (I) in saline as a bolus containing about 1 to about 40 mg/kg of the active ingedient. Desirable blood levels may be maintained by a continuous infusion to provide about 0.01 to about 0.4 mg/kg/hour or by intermittent infusions containing about 0.4 to about 10 mg/kg of the active ingredient.

While it is possible for the active ingedients to be administered alone it is preferable to present them as pharmaceutical formulations. The formulations of the present invention comprise at least one active ingredient, as above defined, together with one or more acceptable carriers therefor and optionally other therapeutic ingredients. The carrier(s) must be "acceptable" in the sense of being compatible with the other ingredients of the formulation and not deleterious to the recipient thereof.

The formulations include those suitable for oral, rectal, nasal, topical (including buccal and sublingual), vaginal or parenteral (including subcutaneous, intramuscular, intravenous and intradermal) administration. The formulations may conveniently be presented in unit dosage form and may be prepared by any of the methods well known in the art of pharmacy. Such methods include the step of bringing into association the active ingredient with the carrier which constitutes one or more accessory ingredients. In general, the formulations are prepared by uniformly and intimately bringing into association the active ingredient with liquid carriers or finely divided solid carriers or both, and then, if necessary, shaping the product.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or molding, optionally with one or more accessory ingredients. Compressed tablets may be prepared by compressing in a suitable machine the active ingredient in a free-flowing form such as a powder or granules, optionally mixed with a binder, lubricant, inert diluent, preservative, surface active or dispersing agent. Molded tablets may be made by molding in a suitable machine a mixture of the powdered compound moistened with an inert liquid diluent. The tablets may optionally be coated or scored and may be formulated so as to provide slow or controlled release of the active ingredient therein.

Topical ointments or creams contain the active ingredient in an amount of, for example, 0.5 to 20% w/w, preferably 1 to 15% w/w and most preferably 2 to 10% w/w. When formulated in an ointment, the active ingredients may be employed with either a paraffinic or a water-miscible ointment base. Alternatively, the active ingredients may be formulated in a cream base.

Formulations suitable for topical administration in the mouth include lozenges comprising the active ingredient in a flavored basis, usually sucrose and acacia or tragacanth; pastilles comprising the active ingredient in an inert basis such as gelatin and glycerin, or sucrose and acacia; and mouthwash comprising the active ingredient in a suitable liquid carrier.

Formulations for rectal administration may be presented as a suppository with a suitable base comprising for example cocoa butter or a salicylate.

Formulations suitable for nasal administration wherein the carrier is a solid include a coarse powder having a particle size, for example, in the range 20 to 500 microns which is administered in the manner in which snuff is taken, i.e. by rapid inhalation through the nasal passage from a container of the powder held close up to the nose. Suitable formulations wherein the carrier is a liquid, for administration as for example a nasal spray or as nasal drops, include aqueous or oily solutions of the active ingredient.

Formulations suitable for vaginal administration may be presented as pessaries, tampons, creams, gels, pastes, foams or spray formulations containing in addition to the active ingredient such carriers as are known in the art to be appropriate.

Formulations suitable for patenteral administration include aqueous and non-aqueous sterile injection solution which may contain anti-oxidants, buffers, bacteriostats and solutes which render the formulation isotonic with the blood of the intended recipient; and aqueous sterile suspensions which may include suspending agents and thickening agents. The formulations may be presented in unit-dose or multi-dose containers, for example, sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage formulations are those containing a daily dose or unit daily sub-dose, as herein above recited, or an appropriate fraction thereof, of an active ingredient.

It should be understood that in addition to the ingredients particularly mentioned above the formulations of this invention may include other agents conventional in the art having regard to the type of formulation in question, for example, those suitable for oral administraiton may include flavoring agents.

The following Examples are for illustration only and are not intended to restrict the scope of the present invention in any way.

## EXAMPLES

### Example 1 — Tablet

| | |
|---|---|
| Compound of Formula (I) | 100 mg |
| Lactose | 200 mg |
| Starch | 50 mg |
| Polyvinylpyrrolidone | 5 mg |
| Magnesium stearate | 4 mg |
| | 359 mg |

Tablets were prepared from the foregoing ingredients by wet granulation followed by compression.

### Example 2 — Injectable Solution

| | |
|---|---|
| Mono-, di- or tri-sodium salt of compound of Formula (I) | 0.775 g |
| Sterile, pyrogen-free, pH 7 phosphate buffer, q.s. to | 25 ml |

### Example 3

#### Antiviral Activity

The compound of formula (1) in which $R^1$, $R^2$ and $R^3$ each represent a hydrogen atom was tested and found to protect target T cells against the cytopathic effects of HTLV-III at concentrations of 10μM and above.

Example 4

Toxicity

No cytotoxicity was observed at concentrations of the above compound of up to 100μM in FE-10 (mouse modified 3T3) cells.

HDL/OLM/14th January 1986

1. Use of a compound of formula (I)

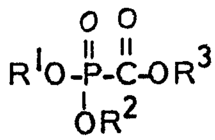

(wherein $R^1$, $R^2$, and $R^3$ may be the same or different and each is selected from hydrogen, $C_{1-6}$ alkyl, $C_{3-6}$ cycloalkyl, $C_{4-7}$ cycloalkyalkyl, benzyl and phenyl having up to three optional substituents selected from halogen, $C_{1-4}$ alkyl, $C_{1-4}$ alkoxy, $C_{2-7}$ alkoxycarbonyl and $C_{2-7}$ alkylcarbonyl and, when at least one of $R^1$, $R^2$ and $R^3$ is hydrogen, pharmaceutically acceptable salts thereof) in the manufacture of a medicament for the treatment or prophylaxis of acquired immune deficiency syndrome.

2. Use of a compound of formula (I) as claimed in claim 1 wherein $R^1$, $R^2$, and $R^3$ each represent hydrogen.

3. Use of a pharmaceutically acceptable salt of a compound of formula (I) as claimed in claim 1 wherein $R^1$, $R^2$, and $R^3$ each represent hydrogen.

4. Use of a compound of formula (I) as claimed in any of claims 1 to 3 wherein the said salt is selected from mono, di- and triphosphate.

5. Use of a compound of formula (I) as claimed in any of claims 1 to 4 wherein the said medicament is adapted for intravenous administration.

6. Use of a compound of formula (I) as claimed in any of claims 1 to 4 wherein the said medicament is in the form of a tablet or capsule.

7. A compound of formula (I) or a pharmaceutically acceptable salt thereof (as defined in any of claims 1 to 4) for use in the treatment or prophylaxis of acquired immune deficiency syndrome.

8. A compound of formula (I) or a pharmaceutically acceptable salt thereof (as defined in any of claims 1 to 4) for use in the treatment or prophylaxis of an HTLV-III infection in a human being.